# FASCICULE DE BREVET EUROPEEN

(11) **EP 2 819 666 B1**
(45) Date de publication et mention de la délivrance du brevet: **17.02.2021**
(21) Numéro de dépôt: 13720036.6
(22) Date de dépôt: 27.02.2013
(51) Int. Cl.: A61K 31/191, A61K 33/06, A61K 9/06, A61K 9/08, A61K 9/12, A61K 47/10, A61L 26/00, A61P 17/02

(54) **FORMULATION GELIFIANTE A BASE DE GLUCONATE DE CALCIUM**
GELIERUNGSFORMULIERUNG AUF BASIS VON CALCIUMGLUCONAT
GELLING FORMULATION BASED ON CALCIUM GLUCONATE

(30) Priorité: 27.02.2012 FR 1251758
(43) Date de publication de la demande: 07.01.2015
(73) Titulaire: Assistance Publique - Hôpitaux de Paris, 75004 Paris (FR)
(72) Inventeur: BOUDY, Vincent, 75013 Paris (FR); GRAFF DE FAGET, Sandrine, 78170 La Celle Saint Cloud (FR); HUSSON, Marie-Caroline, 75005 PARIS (FR); BOUCENNA, Imane, 94700 Maisons-Alfort (FR); ROYON, Laurent, 75005 Paris (FR); COLINART, Pierre, 92140 Clamart (FR)
(74) Mandataire: Gevers & Orès
(86) Numéro de dépôt international: PCT/IB2013/051565
(87) Numéro de publication internationale: WO 2013/128383

(56) Documents cités:
- EP-A1- 0 551 626
- EP-A1- 2 145 617
- EP-A2- 0 134 964
- Orion Laboratories: "calcium gluconate gel 2.5%", , 1 novembre 2011 (2011-11-01), pages 1-3, XP055032874, Extrait de l'Internet: URL:http://www.orion.net.au/products/pdf/c al01466_msds.pdf [extrait le 2012-07-16]
- DEVELAY P ET AL: "Use of a calcium gluconate gel for treatment of skin burns caused by fluorhydric acid", JOURNAL DE PHARMACIE CLINIQUE 1983 FR, vol. 2, no. 2, 1983, pages 115-122, XP009161159, ISSN: 0291-1981
- ROBLIN I ET AL: "Topical treatment of experimental hydrofluoric acid skin burns by 2.5% calcium gluconate", JOURNAL OF BURN CARE AND RESEARCH 200611 US LNKD- DOI:10.1097/01.BCR.0000245767.54278.09, vol. 27, no. 6, novembre 2006 (2006-11), pages 889-894, XP009161161, ISSN: 1559-047X
- HENRY R L ET AL: "BURN WOUND COVERINGS AND THE USE OF POLOXAMER PREPARATIONS", CRITICAL REVIEWS IN BIOCOMPATIBILITY, CRC PRESS, BOCA RATON , FL, US, vol. 5, no. 3, 1 janvier 1989 (1989-01-01) , pages 207-220, XP009031663, ISSN: 0748-5204
- SCHMOLKA I R: "ARTIFICIAL SKIN I. PREPARATION AND PROPERTIES OF PLURONIC F-127 GELS FOR TREATMENT OF BURNS", JOURNAL OF BIOMEDICAL MATERIALS RESEARCH, WILEY, NEW YORK, NY, US, vol. 6, no. 6, 1 novembre 1972 (1972-11-01), pages 571-582, XP009017693, ISSN: 0021-9304, DOI: 10.1002/JBM.820060609
- NALBANDIAN R M ET AL: "PLURONIC F-127 GEL PREPARATION AS AN ARTIFICIAL SKIN IN THE TREATMENT OF THIRD-DEGREE BURNS IN PIGS", JOURNAL OF BIOMEDICAL MATERIALS RESEARCH, WILEY, NEW YORK, NY, US, vol. 21, no. 9, 1 septembre 1987 (1987-09-01), pages 1135-1148, XP002915634, ISSN: 0021-9304, DOI: 10.1002/JBM.820210907

## Description

L'invention concerne une formulation gélifiante à base de gluconate de calcium, son utilisation dans le traitement des brûlures provoquées par l'acide fluorhydrique, et son procédé de préparation.

Le fluorure d'hydrogène (HF) est un liquide fumant incolore ou bien un gaz avec une odeur forte et irritante, extrêmement corrosif. L'acide fluorhydrique est une solution aqueuse de fluorure d'hydrogène. Il s'agit d'un acide fort ayant des effets hautement caustiques et corrosifs sur les tissus organiques, du fait des ions fluorures dissociés (F⁻).

L'acide fluorhydrique est principalement utilisé dans l'industrie comme matière première, notamment dans la fabrication d'acier inoxydable, d'aluminium, de produits chimiques organiques ou inorganiques, dans le raffinage du pétrole, dans la fabrication du verre et de composants électroniques. L'acide fluorhydrique est également présent dans différents produits domestiques (par exemple les produits antirouille). Par conséquent, le risque d'exposition à l'acide fluorhydrique concerne une large population d'individus.

L'acide fluorhydrique peut provoquer des brûlures graves et douloureuses sur la peau et les yeux. Les symptômes, généralement retardés et localisés avec des solutions diluées d'acide fluorhydrique, comprennent des érythèmes, des oedèmes et des douleurs. Comme pour les autres acides, la gravité des brûlures dépend de la concentration et de la température de l'acide fluorhydrique ainsi que de la durée d'exposition. L'acide fluorhydrique se distingue toutefois des autres acides en ce que les ions fluorures pénètrent rapidement la peau, pouvant ainsi causer une destruction des tissus en profondeur, une nécrose des tissus mous, des tendinites, des ténosynovites ou une décalcification de l'os. Contrairement à d'autres acides qui peuvent être rapidement neutralisés, ce processus peut continuer pendant des jours s'il n'est pas traité.

Il existe plusieurs types de traitement des brûlures à l'acide fluorhydriques, parmi lesquels on peut citer notamment l'application topique d'une pâte à base d'oxyde de magnésium^{[1]} ou bien l'injection de composés à base de magnésium tels que le sulfate de magnésium ou l'acétate de magnésium^{[2]}.

D'autres traitements impliquent notamment l'utilisation de composés à base d'ammonium quaternaire^{[3]} tels que la solution de chlorure de benzethonium (Hyamine®)^{[3,4]} ou le chlorure de benzalkonium (Zephiran®)^{[3,4]}. Toutefois, le traitement à l'Hyamine® requière de saturer la zone de brûlure pendant une période prolongée.

Les infiltrations à base de gluconate de calcium sont depuis longtemps utilisées pour traiter efficacement les brûlures à l'acide fluorhydrique^{[1, 2, 4-7]}. Elles permettent de restreindre le degré d'œdème ou de nécrose par l'acide fluorhydrique et empêchent les ions fluorures de pénétrer en profondeur. Ce traitement lourd et invasif est toutefois généralement réservé aux cas de brûlures particulièrement graves, et n'est pas adapté pour prodiguer les premiers soins.

Le gel à base de gluconate de calcium^{[8-12]}, notamment à base de 2,5% de gluconate de calcium, est par ailleurs largement utilisé en premiers soins des brûlures à l'acide fluorhydrique. Après rinçage à l'eau froide, le gel est appliqué sur la peau pour éviter que la blessure ne s'étende. Toutefois, du fait d'une demande assez limitée, le gel à base de gluconate de calcium n'est pas disponible commercialement auprès des laboratoires pharmaceutiques. En France, ce gel également disponible sous forme de préparation magistrale, a le statut règlementaire de préparation hospitalière. Un gel prêt à l'emploi à base de carboxyméthylcellulose/glycérol est ainsi fabriqué et distribué par l'Agence Générale des Produits de Santé (AGEPS, Paris, France). Aux Etats-Unis, ce gel, qui n'a pas reçu l'approbation de l'Agence du Médicament (Food and Drug Administration, FDA), est préparé de façon extemporanée en mélangeant le gluconate de calcium en solution ou sous forme de poudre dans un lubrifiant soluble dans l'eau. Toutefois, l'absence de standardisation de la préparation du gel, ainsi que l'absence de tests de stabilité et de stérilité représentent des facteurs limitants importants pour ces produits non commerciaux. Par ailleurs, ces formulations de type gel ne permettent pas une répartition large du traitement. En outre, cette répartition s'effectue généralement par la main ou au moyen d'une spatule non stérile, ce qui pose des risques de contamination microbienne.

Il a maintenant été mis au point une nouvelle formulation permettant de pallier aux inconvénients des traitements actuels, et notamment du gel à base de gluconate de calcium. Plus particulièrement, l'invention concerne une formulation à base de gluconate de calcium, capable de façon avantageuse, d'être pulvérisée sous forme liquide sur la brûlure et de se gélifier instantanément au contact de la peau. Comparé à une solution, la présence d'une texture gel à la surface de la peau permet un contact prolongé du gluconate de calcium avec la blessure. Cette formulation offre plusieurs avantages : elle est tout d'abord facile à appliquer et peut être répartie de façon large sur les brûlures à traiter ; elle peut être par ailleurs aisément préparée et conditionnée pour être conservée dans des conditions stériles ; cette formulation est particulièrement stable dans le temps et peut être conservée pendant plusieurs mois. Enfin, cette formulation permet de prodiguer des premiers soins de façon rapide, sûre et économique dans des cas de brûlures accidentelles à l'acide fluorhydrique.

L'objet de la présente invention est défini par les revendications 1 à 9.

Ainsi, selon un premier aspect, la description concerne une formulation gélifiante comprenant du gluconate de calcium en solution dans l'eau et un poloxamer.

Par « formulation gélifiante », on entend, au sens de la présente description, une formulation capable de se gélifier, en particulier dans laquelle la concentration du ou des poloxamers est suffisante pour permettre à la solution aqueuse de se gélifier à une température donnée, notamment au contact de la peau.

Le gluconate de calcium est le composé de structure suivante :

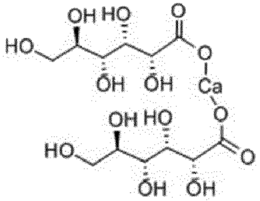

L'avidité des ions fluorures pour les ions calcium pour former des sels insolubles est à la base des traitements des brûlures à l'acide fluorhydrique par le gluconate de calcium.

Les formulations selon la présente demande comprennent de préférence de 1 à 5 % en poids de gluconate de calcium, plus préférentiellement environ 2,5 % en poids par rapport au poids total de la formulation.

Tel qu'on l'emploie ici, le terme « poloxamer » désigne un copolymère bloc comprenant ou consistant en une chaîne centrale polyoxypropylène (encore appelé polypropylène glycol, POP) greffée de part et d'autre par une chaîne polyoxyéthylène (encore appelé polyéthylène glycol, POE). Les poloxamers sont généralement désignés par la lettre « P » (pour poloxamer) suivie par trois chiffres : les deux premiers chiffres multipliés par 100 donnent la masse moléculaire du cœur polyoxypropylène, et le dernier chiffre multiplié par 10 donne le pourcentage de la teneur en polyoxéthylène. A titre d'exemple, P407 correspond à un poloxamer dont le cœur en polyoxypropylène a une masse moléculaire de 4000g/mol et une teneur en polyoxyéthylène de 70%.

Les poloxamers utiles selon la description sont des poloxamers thermosensibles, qui sont à l'état liquide à température ambiante, notamment entre 15 et 25°C, et à l'état gel à une température supérieure ou égale à leur température de gélification (T*_{g}*), notamment dans des conditions physiologiques.

Les températures de gélification des poloxamers (T*_{g}*) peuvent être déterminées selon des méthodes conventionnelles ou sont disponibles dans des ouvrages de référence tels que le *Handbook of Pharmaceutical Excipients.*

Plus particulièrement, ces poloxamers sont présents dans une concentration suffisante dans la solution aqueuse de gluconate de calcium pour permettre sa gélification lorsque la température est supérieure ou égale à leur température de gélification (T*_{g}*), notamment dans des conditions physiologiques.

De préférence, la formulation se gélifie à une température comprise entre 27 et 37°C, plus préférentiellement au contact de la peau, notamment à une température comprise entre 31 et 34°C. Les formulations selon la présente demande comprennent généralement de 10 à 30% en poids de poloxamer, de préférence de 10 à 25 % en poids par rapport au poids total de la composition. Comme exemple, on peut citer notamment le poloxamer 407, le poloxamer 188 ou un mélange de ceux-ci.

Selon une variante préférée, les formulations selon la demande comprennent environ 2,5 % en poids de gluconate de calcium et environ 13 à 16 % en poids, notamment environ 15 % en poids de poloxamer 407, par rapport au poids total de la composition. Elles comprennent en outre du poloxamer 188, en une quantité pouvant varier notamment de 1 à 10 % en poids, en particulier de 1 à 6% en poids par rapport au poids total de la composition.

Les formulations selon la présente demande peuvent comprendre en outre des excipients, notamment des agents de solubilisation du gluconate de calcium tels que le propylène glycol ou des sels tels que le chlorure de sodium, ou encore des agents de bioadhésion tels que l'hydroxypropylméthylcellulose, la méthylcellulose ou les polymères d'acide acrylique réticulés.

Les formulations selon l'invention comprennent en outre du chlorure de sodium, en particulier de 1 à 6% en poids de chlorure de sodium par rapport au poids total de la composition.

Selon une variante préférée, les formulations de la description comprennent environ 15 % en poids de poloxamer 407, de 0 à 6 % en poids environ de poloxamer 188 et de 0 à 6 % en poids environ de chlorure de sodium, les pourcentages en poids se référant au poids total de la composition.

De manière avantageuse, les formulations selon la description sont stériles et peuvent être préparées par filtration stérilisante, par radiostérilisation, ou bien dans des conditions aseptiques. Plus particulièrement, cette solution peut être préparée et/ou conservée dans des conditions stériles dans un flacon muni d'un pulvérisateur adapté.

Selon un autre aspect, la description concerne une composition pharmaceutique comprenant ou consistant en une formulation gélifiante telle que définie ci-dessus.

Selon encore un autre aspect, la description concerne une formulation gélifiante telle que définie ci-dessus pour son utilisation dans le traitement des brûlures cutanées provoquées par l'acide fluorhydrique, destinée notamment à être administrée par voie topique, sur la brûlure.

De façon préférée, la formulation selon la présente demande est pulvérisée sur la peau, et forme instantanément un gel sur la zone d'application.

Selon un autre aspect, la description concerne un procédé de préparation d'une formulation gélifiante telle que définie ci-dessus, ledit procédé comprenant les étapes de :
i) Dissolution à froid du poloxamer dans l'eau ;
ii) Ajout et dissolution du gluconate de calcium dans la solution obtenue à l'étape i) préalablement portée à une température comprise entre 60°C et 90°C ; et éventuellement
iii) Récupération de la formulation obtenue.

Selon une alternative, le procédé comprend les étapes de :
i) Dissolution du gluconate de calcium dans l'eau à une température comprise entre 60°C et 90°C ; et
ii) Ajout et dissolution du poloxamer dans la solution obtenue à l'étape i) préalablement refroidie, notamment à une température comprise entre 15 et 25°C ; et éventuellement
iii) Récupération de la formulation obtenue.

### Définitions

Tel qu'on l'utilise dans la présente description, le terme « environ » se réfère à un intervalle de valeurs de ± 10 % d'une valeur spécifique. A titre d'exemple, l'expression « 16 % environ » comprend les valeurs de 16 % ± 10 %, soit les valeurs de 14,4 % à 17,6 %.

Au sens de la présente description, les pourcentages se réfèrent à des pourcentages en poids par rapport au poids total de la formulation, sauf indication contraire.

Tel qu'on l'entend ici, les plages de valeur sous forme de « x-y » ou « de x à y » ou « entre x et y » incluent les bornes x et y ainsi que les entiers compris entre ces bornes. A titre d'exemple, « 1-6 », ou « de 1 à 6 » ou « entre 1 et 6 » désignent les entiers 1, 2, 3, 4, 5 et 6. Les modes de réalisations préférés incluent chaque entier pris individuellement dans la plage de valeur, ainsi que toute sous-combinaison de ces entiers. A titre d'exemple, les valeurs préférées pour « 1-6 » peuvent comprendre les entiers 1, 2, 3, 4, 5, 6, 1-2, 1-3, 1-4, 1-5, 2-3, 2-4, 2-5, 2-6, etc.

### EXEMPLES

### Exemple 1: Préparation d'une formulation gélifiante à 2,5% de gluconate de calcium (méthode A)

16 g de poloxamer 407 (Lutrol®, BASF) ont été dissous à froid, *i.e.* à température ambiante, dans 81,5 mL d'eau. La solution obtenue a ensuite été chauffée à 80°C environ puis 2,5 g de gluconate de calcium ont été ajoutés et dissous en agitant pendant une heure environ.

La solution obtenue se gélifie au contact de la peau.

### Exemple 2 : Préparation d'une formulation gélifiante à 2,5% de gluconate de calcium (méthode B)

2,5 g de gluconate de calcium ont été dissous sous agitation dans 81,5 mL d'eau préalablement chauffée à 80°C. La solution obtenue a ensuite été refroidie jusqu'à la température ambiante avant ajout de 16 g de poloxamer 407.

La solution obtenue se gélifie au contact de la peau.

### Exemple 3 :

Les formulations rapportées dans le tableau ci-après ont été préparées selon les méthodes A et B ci-dessus.

L'osmolarité est comprise entre 600 et 1000 mOsm/Kg.

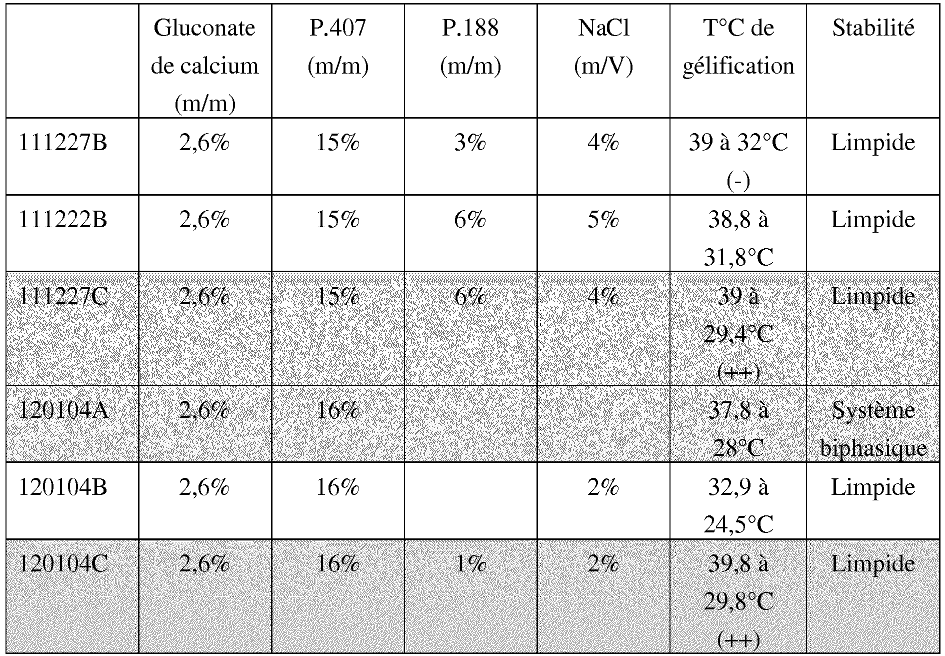

### REFERENCES

[1]. Jones AT., J. Ind. Hyg. Toxicol, 1939, 21: 205.
[2]. Harris JC, Rumack BH, Bregman DJ., Clin. Toxicol, 1981, 18 : 1027-1032.
[3]. Reinhardt CF, Hume WG, Linch AL, Wetherhold JM, Am. Ind. Hyg. Assoc. J., 1966, 27: 166-171.
[4]. Dibbell DG, Iverson RE, Jones WJ, Laub DR, Madison MS, J. Bone Joint Surg., 1970, 52 : 931-936.
[5]. Paley and Seifter, Proc. Soc. Exp. Biol. Med., 1941, 46 : 190.
[6]. Iverson RE, Laub DR, Madison MS, Plast. Reconstr. Surg., 1971, 48 : 107-112.
[7]. Velvart J., Hum. Toxicol, 1983, 2 : 233-238.
[8]. Browne TD, J. Soc. Occup. Med., 1974, 24 : 80-89.
[9]. Bracken WM, Cuppage F, McLaury RL, Kirwin C, Klassen CD, J. Occup. Med., 1985, 27: 733-739.
[10]. Kodoma Y, Matsuno K, Kayama F, Suenaga R, Jpn J. Ind. Health, 1988, 30 : 400-401.
[11]. Kono K, Yoshida Y, Watanabe M, et al., Recent advances in researches on the combined effects of environmental factors, Kasuya M ed, Toyama Japan: Chuetsu Co Ltd, 1994: 407-413.
[12]. Roblin I., Urban M, Flicoteau D., Martin C., Pradeau D., J. Burn. Care Res., 2006 Nov-Dec ; 27(6) : 889-94.

## Revendications

1. Formulation gélifiante comprenant du gluconate de calcium en solution dans l'eau, du poloxamer 407, du poloxamer 188 et du chlorure de sodium.

2. Formulation gélifiante selon la revendication 1, comprenant de 1 à 5 % en poids de gluconate de calcium, de préférence environ 2,5 % en poids par rapport au poids total de la formulation.

3. Formulation gélifiante selon l'une quelconque des revendications précédentes, comprenant de 10 à 30 % en poids de poloxamer, de préférence de 10 à 25 % en poids par rapport au poids total de la formulation.

4. Formulation gélifiante selon l'une quelconque des revendications précédentes, **caractérisée en ce qu'**elle est stérile.

5. Composition pharmaceutique comprenant ou consistant en une formulation gélifiante selon l'une quelconque des revendications 1 à 4.

6. Formulation gélifiante selon l'une quelconque des revendications 1 à 4, pour son utilisation dans le traitement des brûlures cutanées provoquées par l'acide fluorhydrique.

7. Formulation gélifiante pour son utilisation selon la revendication 6, **caractérisée en ce qu'**elle est pulvérisée sur la peau.

8. Procédé de préparation d'une solution gélifiante selon l'une quelconque des revendications 1 à 4 précédentes, ledit procédé comprenant les étapes de :
i) Dissolution à froid du poloxamer 407 et du poloxamer 188 dans l'eau ;
ii) Ajout et dissolution du gluconate de calcium dans la solution obtenue à l'étape i) portée à une température comprise entre 60°C et 90°C .

9. Procédé de préparation d'une solution gélifiante selon l'une quelconque des revendications 1 à 4 précédentes, ledit procédé comprenant les étapes de :
i) Dissolution du gluconate de calcium dans l'eau à une température comprise entre 60°C et 90°C ; et
ii) Ajout et dissolution du poloxamer 407 et du poloxamer 188 dans la solution obtenue à l'étape i) préalablement refroidie.

## Patentansprüche

1. Gelierungsformulierung, umfassend Calciumgluconat als Lösung im Wasser, Poloxamer 407, Poloxamer 188 und Natriumchlorid.

2. Gelierungsformulierung nach Anspruch 1, umfassend von 1 bis 5 Gew.-% an Calciumgluconat, vorzugsweise etwa 2,5 Gew.-% in Bezug auf das Gesamtgewicht der Formulierung.

3. Gelierungsformulierung nach einem der vorstehenden Ansprüche, umfassend von 10 bis 30 Gew.-% an Poloxamer, vorzugsweise 10 bis 25 Gew.-% in Bezug auf das Gesamtgewicht der Formulierung.

4. Gelierungsformulierung nach einem der vorstehenden Ansprüche, **dadurch gekennzeichnet, dass** sie steril ist.

5. Pharmazeutische Zusammensetzung, umfassend oder bestehend aus einer Gelierungsformulierung nach einem der Ansprüche 1 bis 4.

6. Gelierungsformulierung nach einem der Ansprüche 1 bis 4 zu deren Verwendung bei der Behandlung von durch Flusssäure hervorgerufenen Hautverbrennungen.

7. Gelierungsformulierung zu deren Verwendung nach Anspruch 6, **dadurch gekennzeichnet, dass** sie auf die Haut gesprüht wird.

8. Verfahren zur Herstellung einer Gelierungsformulierung nach einem der vorstehenden Ansprüche 1 bis 4, wobei das Verfahren die folgenden Schritte umfasst:
i) Auflösen des Poloxamer 407 und des Poloxamer 188 in kaltem Wasser;
ii) Beigeben und Auflösen des Calciumgluconats in die in Schritt i) erhaltene Lösung, die auf eine Temperatur gebracht wird, die zwischen 60 °C und 90 °C enthalten ist.

9. Verfahren zur Herstellung einer Gelierungsformulierung nach einem der vorstehenden Ansprüche 1 bis 4, wobei das Verfahren die folgenden Schritte umfasst:
i) Auflösen des Calciumgluconats im Wasser bei einer Temperatur, die zwischen 60 °C und 90 °C enthalten ist; und
ii) Beigeben und Auflösen des Poloxamer 407 und des Poloxamer 188 in die in Schritt i) erhaltene, zuvor abgekühlte Lösung.

## Claims

1. Gelling formulation comprising calcium gluconate in solution in water, Poloxamer 407, Poloxamer 188, and sodium chloride.

2. Gelling formulation according to claim 1, comprising between 1 and 5% by weight of calcium gluconate, preferably approximately 2.5% by weight in ratio to the total weight of the formulation.

3. Gelling formulation according to any one of the preceding claims, comprising from 10 to 30% by weight of Poloxamer, preferably from 10 to 25% by weight in ratio to the total weight of the formulation.

4. Gelling formulation according to any one of the preceding claims, **characterised in that** it is sterile.

5. Pharmaceutical composition comprising or consisting of a gelling formulation according to any one of the claims 1 to 4.

6. Gelling formulation according to any one of the claims 1 to 4, for use in the treatment of skin burns caused by hydrofluoric acid.

7. Gelling formulation for use according to claim 6, **characterised in that** it is sprayed on the skin.

8. Method of preparation of a gelling solution according to any of the preceding claims 1 to 4, said method comprising the following steps:
i) cold dissolution of the Poloxamer 407 and the Poloxamer 188 in water;
ii) adding and dissolution of the calcium gluconate in the solution obtained during step i) brought to a temperature of between 60° C and 90° C.

9. Method of preparation of a gelling solution according to any one of the preceding claims 1 to 4, said method comprising the following steps:
i) dissolution of the calcium gluconate in water at a temperature of between 60° C and 90° C; and
ii) adding and dissolution of the Poloxamer 407 and the Poloxamer 188 in the solution obtained in step i), pre-cooled.
